# EUROPÄISCHE PATENTSCHRIFT

(11) **EP 2 459 512 B1**
(45) Veröffentlichungstag und Bekanntmachung des Hinweises auf die Patenterteilung: **17.08.2016**
(21) Anmeldenummer: 10736987.8
(22) Anmeldetag: 20.07.2010
(51) Int. Cl.: C07C 209/08, C07C 211/15

(54) **VERFAHREN ZUR HERSTELLUNG VON 2,2-DIFLUORETHYLAMIN**
METHOD FOR MANUFACTURING 2,2-DIFLUORETHYLAMIN
PROCÉDÉ DE FABRICATION DE 2,2-DIFLUORÉTHYLAMINE

(30) Priorität: 28.07.2009 EP 09166574
(43) Veröffentlichungstag der Anmeldung: 06.06.2012
(73) Patentinhaber: Bayer Intellectual Property GmbH, 40789 Monheim am Rhein (DE)
(72) Erfinder: LUI, Norbert, 51519 Odenthal (DE); PAZENOK, Sergii, 42699 Solingen (DE); SHERMOLOVICH, Yuriy, Grigorievich, 252039 Kiew (UA)
(74) Vertreter: BIP Patents
(86) Internationale Anmeldenummer: PCT/EP2010/004434
(87) Internationale Veröffentlichungsnummer: WO 2011/012243

(56) Entgegenhaltungen:
- SWARTS: "Über einige fluorhaltige Alkylamine" CHEM ZENTRALBL, Bd. 75, 1904, Seiten 944-945, XP009126801 in der Anmeldung erwähnt
- DICKEY ET AL.: "Fluorinated Aminoanthraquinone dyes" INDUSTRIAL AND ENGINEERING CHEMISTRY, Bd. 48, Nr. 2, 1956, Seiten 209-213, XP002559402 in der Anmeldung erwähnt

## Beschreibung

Die vorliegende Erfindung betrifft ein Verfahren zur Herstellung von 2,2-Difluorethylamin ausgehend von 2,2-Difluor-1-halogenethan.

2,2-Difluorethylamin ist eine wichtige Zwischenverbindung bei der Wirkstoffherstellung. Es sind verschiedene Herstellungsmethoden für 2,2-Difluorethylamin bekannt.

Donetti et al. (J. Med. Chem. 1989, 32, 957-961) beschreiben die Synthese von 2,2-Difluorethylamin-Hydrochlorid ausgehend von 2,2-Difluoracetamid. Hierbei wird mit einer Diboran-Lösung in Tetrahydrofuran (THF) das gewünschte Amin hergestellt. Die Ausbeute beträgt 48%.

Kluger et al. (JACS 1982, 104, 10, 2891-2897) beschreiben die Synthese von 2,2-Difluorethylamin ausgehend vom Amid mit Natriumboranat und Bortrifluoridetherat. Die Ausbeute beträgt 60%.

Auch Kollonitsch (US 4,030,994) beschreibt eine Synthese von 2,2-Difluorethylamin, nämlich die Umsetzung von Ethylamin mit Fluoroxytrifluoromethan in Fluorwasserstoff unter UV Bestrahlung.

Swarts beschreibt in seinem Artikel mit dem Titel "Über einige fluorhaltige Alkylamine" (Chem. Zentralblatt, Band 75, 1904, Seiten 944-945) die Herstellung von 2,2-Difluorethylamin und Tetrafluorethylamin, mit anschließender Abtrennung der beiden Produkte durch fraktionierte Destillation oder durch Umwandlung derselben in Chlorhydrate oder Oxalate. Swarts verwendet 1-Brom-2,2-difluorethan und erhitzt dieses 3 Tage lang im Rohr mit 2 Mol alkoholischem Ammoniak auf 125- 145 °C. Swarts beschreibt die vollständige Umwandlung der Ausgangsverbindung in die Verbindungen Difluorethylamin und Tetrafluorethylamin.

Die Herstellung von 2,2-Difluorethylamin wird auch bei Dickey et al. (Industrial and Engineering Chemistry 1956, Nr. 2, 209- 213) beschrieben. 2,2-Difluor-1-chlorethan wird dort mit 28 %-igem Ammoniumhydroxid, d.h. 28 %-iger wässriger Ammoniak-Lösung, in einem Autoklaven (rocking autoclave) zur Reaktion gebracht. Die Reaktionsmischung wird für 31 Stunden auf Temperaturen von 135° bis 140 °C erhitzt. Nach Beendigung der Reaktion wird die Reaktionsmischung filtriert und das Amin vom Reaktionsgemisch abdestilliert. Da sich aber noch eine Menge Ammoniak und etwas Wasser im Destillat befindet, wird das Amin über Natriumhydroxid getrocknet und nochmals destilliert. Das Amin wurde so in einer Ausbeute von 65 % gewonnen.

Dieses Verfahren ist nachteilig, denn es benötigt - genauso wie das Verfahren nach Swarts - eine sehr lange Reaktionszeit von 31 Stunden und die Ausbeute von 65 % ist eher gering. Gleichzeitig ist die Reaktionsmischung stark korrodierend, da der wässrige Ammoniak in Kombination mit den im Reaktionsgemisch vorhandenen Chlorid- und Fluoridionen bei den im Verfahren verwendeten hohen Temperaturen metallische Werkstoffe angreift.

Alle diese bekannten Verfahren sind nachteilig, insbesondere weil sie sich nicht im wirtschaftlich sinnvollen großtechnischen (industriellen) Maßstab durchführen lassen. Die geringe Ausbeute und der Einsatz von teuren und gefährlichen Chemikalien wie z.B. Natriumboranat/BF₃ oder Diboran verhindern, dass die Verfahren nach Donetti et al. und Kluger et al. für die großtechnische Herstellung von 2,2-Difluorethylamin geeignet sind. Das Verfahren nach Kollonitsch et al. verwendet gefährliche Chemikalien und es wird kein reines 2,2-Difluorethylamin erhalten. Das Verfahren nach Dickey et. al. und das Verfahren nach Swarts sind ebenfalls für den großtechnischen Einsatz ungeeignet bzw. unwirtschaftlich, denn sie benötigen sehr lange Reaktionszeiten und sind gleichzeitig nicht selektiv, so dass die Ausbeuten der Verfahren unbefriedigend.

Ausgehend von den bekannten Verfahren zur Herstellung von 2,2-Difluorethylamin stellt sich nun die Aufgabe, wie 2,2-Difluorethylamin einfach und kostengünstig hergestellt werden kann. Unter kostengünstigen Verfahren werden solche Verfahren verstanden, die ohne großen finanziellen Aufwand durchzuführen sind, weil die Ausgangsstoffe beispielsweise ungefährlich sind, keine sonstigen technischen Probleme auftauchen, beispielsweise weil das Reaktionsgemisch korrodierend wirkt, und/oder das gewünschte 2,2-Difluorethylamin in einer ausreichend hohen Ausbeute und Reinheit erhalten wird, weil etwa die Reaktion weitgehend selektiv verläuft.

Ausgehend von 2,2-Difluor-1-halogenethan wurde nun ein Verfahren zur Herstellung von 2,2-Difluorethylamin gefunden, dass einfach und kostengünstig durchführbar ist, insbesondere, weil der Ausgangsstoff 2,2-Difluor-1-halogenethan schon unter vergleichsweise milden Reaktionsbedingungen und kurzen Reaktionszeiten selektiv zum gewünschten 2,2-Difluorethylamin reagiert.

Gegenstand der Erfindung ist somit ein Verfahren zur Herstellung von 2,2-Difluorethylamin, umfassend die Umsetzung von 2,2-Difluor-1-halogenethan der folgenden allgemeinen Formel (I):

CHF₂-CH₂Hal,

wobei Hal für Cl, Br, oder Jod steht, mit NH₃ (Ammoniak) in einem Lösungsmittel das einen Wassergehalt von maximal 0,5 Vol.-% aufweist und in Gegenwart eines die Reaktion mit Ammoniak beschleunigenden Katalysators, wobei der Katalysator ausgewählt ist aus der Gruppe bestehend aus Alkalibromiden und -jodiden, Ammoniumbromid und Ammoniumjodid, Tetraalkylammoniumbromiden und -jodiden, gegebenenfalls mit anschließender Aufreinigung, vorzugsweise destillativer Aufreinigung. 2,2-Difluor-1-halogenethan der allgemeinen Formel (I) in denen Hal für Chlor und Brom steht ist bevorzugt. Ganz bevorzugt ist die Verbindung CHF₂-CH₂Cl (2,2-Difluor-1-chlorethan).

Das molare Verhältnis von 2,2-Difluor-1-halogenethan zum eingesetzten Ammoniak NH₃ liegt im Bereich von 0,8 : 1 bis 1 : 30, bevorzugt im Bereich von etwa 1 : 2 bis etwa 1 : 20, besonders bevorzugt im Bereich von etwa 1 : 3 bis etwa 1 : 12.

Erfindungsgemäß werden Lösungsmittel verwendet, die einen Wassergehalt von maximal 0,5 Vol-% aufweisen.

Lösungsmittel werden allgemein in einer solchen Menge eingesetzt, dass das Reaktionsgemisch während des ganzen Verfahrens gut rührbar ist. Vorteilhafterweise wird, bezogen auf das eingesetzte 2,2-Difluor-1-halogenethan, die 1 bis 50-fache Lösungsmittelmenge, bevorzugt die 2 bis 40-fache Lösungsmittelmenge, besonders bevorzugt die 2 bis 20-fache Lösungsmittelmenge verwendet.

Als Lösungsmittel zur Durchführung des erfindungsgemäßen Verfahrens kommen alle unter den Reaktionsbedingungen inerten organischen Lösungsmittel in Frage. Unter Lösungsmittel werden erfindungsgemäß auch Gemische reiner Lösungsmittel verstanden.

Erfindungsgemäß geeignete Lösungsmittel sind insbesondere Alkohole (z.B. Methanol, Ethanol, Isopropanol, Butanol (d.h. n-Butanol, tert-Butanol, 2-Butanol), 2-(2-Ethoxyethoxy)-ethanol, Diethylenglykol); Ether (z.B. Ethylpropylether, *n*-butylether, Anisol, Phenetol, Cyclohexylmethylether, Dimethylether, Diethylether, Dimethylglycol, Diphenylether, Diproplether, Diisopropylether, Di-*n*-butylether, Diisobutylether, Diisoamylether, Ethylenglycoldimethylether, Isopropylethylether, Methyl-tert-butylether, Diethylenglykoldimethylether, Triethylengylkoldimethylether, Tetrahydrofuran, Dioxan, und Polyether des Ethylenoxids und/oder Propylenoxids); Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Octan, Benzol, Toluol, Chlorbenzol, Brombenzol, Xylol); Ester (z.B. Methyl-, Ethyl-, Butyl-, Isobutylacetat, Dimethyl-, Dibutyl- oder Ethylencarbonat, Propylencarbonat); Amide (z.B. Hexamethylenphosphorsäuretriamid, Formamid, N,N-Dimethylacetamid, *N*-Methyl-formamid, *N,N*-Dimethyl-formamid, *N,N*-Dipropyl-formamid, *N,N*-Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N*'-1,4-Diformyl-piperazin) oder Gemische davon.

Im erfindungsgemäßen Verfahren werden als Lösungsmittel bevorzugt Alkohole, insbesondere n-Butanol, Amide, insbesondere N-Methylpyrrolidin oder 1,3-Dimethyl-2-imidazolindion, Ether, insbesondere Triethylenglykoldimethylether, sowie Dimethylsulfoxid oder Tetramethylensulfoxid oder Gemische davon eingesetzt.

Für die Verwendung im erfindungsgemäßen Verfahren sind alle Katalysatoren geeignet, die die Umsetzung mit Ammoniak beschleunigen. Mischungen geeigneter Katalysatoren sind auch denkbar. Erfindungsgemäß geeignet sind Alkalibromide und -jodide (z.B. Natriumjodid, Kaliumjodid, Kaliumbromid); Ammoniumbromid und Ammoniumjodid; Tetraalkylammoniumbromide und -jodide, (z.B. Tetraethylammoniumjodid); bestimmte

Im erfindungsgemäßen Verfahren werden als Katalysatoren bevorzugt Natriumjodid, Kaliumjodid oder Tetrabutylammoniumbromid, besonders bevorzugt Natrium- oder Kaliumjodid eingesetzt.

Der Katalysator kann auch *in-situ* erzeugt werden, beispielsweise durch eine Reaktion von HBr oder HJ mit Ammoniak. Weiterhin kann der Katalysator auch *in-situ* durch Zugabe von sehr reaktiven Alkylbromiden oder -jodiden (z.B. Methyl- oder Ethylbromid oder -jodid) erzeugt werden.

Im erfindungsgemäßen Verfahren wird der Katalysator, bezogen auf das eingesetzte 2,2-Difluor-1-halogenethan, in einer Konzentration von etwa 0,01 bis etwa 25 Gew.-% verwendet. Höhere Konzentrationen sind grundsätzlich möglich. Bevorzugt wird der Katalysator in einer Konzentration von etwa 0,2 bis etwa 25 Gew.-%, besonders bevorzugt von etwa 0,4 bis etwa 20 Gew.-%, ganz besonders bevorzugt von etwa 0,5 bis etwa 15 Gew.-%. verwendet. Der Katalysator kann aber auch bevorzugt in einer Konzentration von etwa 0,05 bis etwa 3 Gew.-%, von etwa 0,1 bis etwa 10 Gew.-% oder von etwa 0,5 bis etwa 10 Gew.-% eingesetzt werden.

Die Erfinder haben festgestellt, dass die Selektivität und damit die Ausbeute sowie die Reaktionsgeschwindigkeit der Umsetzung durch den Einsatz eines die Umsetzung mit Ammoniak beschleunigenden Katalysators und durch das Verwenden von Lösungsmittel mit einem maximalen Wassergehalt von 15 Vol.-% sehr vorteilhaft sind und die Grundlage dafür bilden, dass die Umsetzung im großtechnischen Maßstab durchgeführt werden kann. Auch ist das Reaktionsgemisch durch das Verwenden eines Lösungsmittels mit einem Wassergehalt von maximal 15 Vol.-% weniger korrosiv, weil weniger Fluor oder Halogen-Ionen aus dem 2,2-Difluor-1-halogenethan eliminiert werden.

Die erfindungsgemäße Umsetzung kann in einem weiten Temperaturbereich (z.B. im Bereich von etwa 50 °C bis etwa 200 °C) durchgeführt werden. Vorzugsweise wird die Umsetzung in einem Temperaturbereich von etwa 80 °C bis etwa 160 °C durchgeführt.

Die Umsetzung wird grundsätzlich unter Eigendruck in einem druckstabilen geschlossenen Versuchsgefäß (Autoklav) durchgeführt. Der Druck während der Reaktion (d.h. der Eigendruck) ist abhängig von der verwendeten Reaktionstemperatur, dem verwendeten Lösungsmittel, dem verwendeten 2,2-Difluor-1-halogenethan und der Menge an verwendetem Ammoniak. Ist eine Druckerhöhung gewünscht, so kann eine zusätzliche Druckerhöhung mittels Zugabe eines Inertgases, wie Stickstoff oder Argon, durchgeführt werden.

Die Reaktionsdauer der Reaktion ist kurz und liegt im Bereich von etwa 0,5 bis etwa 10 Stunden. Eine längere Reaktionsdauer ist möglich, jedoch wirtschaftlich nicht sinnvoll.

Wie bereits erwähnt wird das gewünschte 2,2-Difluorethylamin mit dem erfindungsgemäßen Verfahren mit guten Ausbeuten, kurzen Reaktionszeiten und in hoher Reinheit erhalten, sodass eine umfangreiche Aufarbeitung des unmittelbaren Reaktionsprodukts im Allgemeinen nicht erforderlich ist. All das ist überraschend, denn aus den M. Hudlicky, Chemistry of Organofluorine Compounds, 2nd Edition, 1976, p. 489-490 und Houben Weyl, E 10b/2 p. 92-98 ist bekannt, dass unter basischen Bedingungen sich bevorzugt das Vinylidenfluorid bildet, das unter Abspaltung von HCl, HBr oder HJ aus 2,2-Difluorhalogenethan entstehen kann. Auch ist aus J. Org. Chem. 2007, 72 (22) 8569 bekannt, dass 2,2-Difluorethylamin sehr reaktiv ist und unter den erfindungsgemäßen Reaktionsbedingungen weiterreagieren kann.

Die Aufarbeitung des Reaktionsgemisches sowie die Aufreinigung kann über 2,2-Difluorethylamin oder über seine Salze erfolgen. Normalerweise wird der nicht umgesetzte Ammoniak, vorzugsweise durch Destillation, rückgewonnen und die gegebenenfalls vorhandenen Ammoniumsalze abfiltriert. Das 2,2-Difluorethylamin kann dann unter Normaldruck oder im Vakuum aus dem Reaktionsgemisch, vorzugsweise durch Destillation, isoliert werden.

Die Aufreinigung eines 2,2-Difluorethylamin-Salzes, beispielsweise Salze organischer oder anorganischer Säuren (z.B. Hydrochloride oder Acetate), erfolgt bevorzugt durch Kristallisation. 2,2-Difluorethylamin-Salze sind z.B. das 2,2-Difluorethylamin-Hydrochlorid oder 2,2-Difluorethylamin-Acetat. Wasserlösliche Salze können durch Extraktion der wässrigen Lösungen gereinigt werden. Das Amin kann dann schließlich durch Umsetzung mit organischen oder anorganischen Basen aus seinen Salzen freigesetzt werden. Bevorzugte Basen sind NaHCO₃, Na₂CO₃ oder NaOH.

Die vorliegende Erfindung wird anhand der nachfolgenden Beispiele näher erläutert.

### Herstellungsbeispiele:

### Beispiel 1

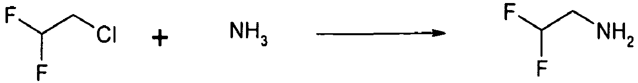

15g (0,149 mol) 2,2-Difluor-1-chlorethan, 1,5g Kaliumjodid werden in einem Autoklaven mit 30g N-Methylpyrrolidin, das einen Wassergehalt von 100 ppm, d.h. 0,01 Vol.%, aufweist, vorgelegt und mit 20,2g Ammoniak versetzt. Das molare Verhältnis von 2,2-Difluor-1-chlorethan zu Ammoniak beträgt 1 : 8. Die Reaktionsmischung wird auf 143°-145°C erhitzt und 5,5 Stunden bei dieser Temperatur gerührt. Die Reaktionsmischung wird auf 50°C abgekühlt und der überschüssige Ammoniak abdestilliert. Das ausgefallene Ammoniumsalz wird abfiltriert und mit 5g N-Methylpyrrolidin gewaschen. Aus der Mutterlauge und Waschlösung wird anschließend bei 1-10 mbar das freie Difluorethylamin herausdestilliert. Man erhält 88% Ausbeute.
NMR ¹H (CDCl₃ 5,5 - 5,9 (m, 1H), 2,94 - 3,1 (m, 2 H), 1,26 (br m, NH₂)

### Beispiel 2

25g (0,249 mol) 2,2-Difluor-1-chlorethan, 2,5g Kaliumjodid werden in einem Autoklaven mit 250g Dimethylsulfoxid (DMSO), das einen Wassergehalt von 150 ppm, d.h. 0,015 Vol.-%, aufweist, vorgelegt und mit 25,4g Ammoniak versetzt. Das molare Verhältnis von 2,2-Difluor-1-chlorethan zu Ammoniak beträgt 1 : 6. Die Reaktionsmischung wird auf 143-145°C erhitzt und 4,5 Stunden bei dieser Temperatur gerührt. Die Reaktionsmischung wird auf 50°C abgekühlt und der überschüssige Ammoniak abdestilliert. Aus der Reaktionsmischung wird das Difluorethylamin anschließend bei 1-10 mbar herausdestilliert. Das Destillat, das noch geringe Mengen an Lösungsmittel enthält wird dann bei Normaldruck redestilliert. Man erhält 87% Ausbeute.

### Beispiel 3

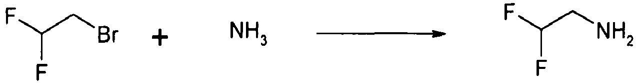

14,5g (0,1 mol) 2,2-Difluor-1-bromethan, 0,5g Kaliumjodid werden in einem Autoklaven mit 50g DMSO, das einen Wassergehalt von 250 ppm, d.h. 0,025 Vol.%, aufweist, vorgelegt und mit 6,8g wasserfreien Ammoniak versetzt. Das molare Verhältnis von 2,2-Difluor-1-bromethan zu Ammoniak beträgt 1 : 4. Die Reaktionsmischung wird auf 100°C erhitzt und 1 Stunde bei dieser Temperatur gerührt. Die Reaktionsmischung wird auf 50°C abgekühlt und der überschüssige Ammoniak abdestilliert. Aus der Reaktionsmischung wird das Difluorethylamin anschließend bei 1-10 mbar herausdestilliert. Das Destillat, das noch geringe Mengen an Lösungsmittel enthält wird dann bei Normaldruck redestilliert. Man erhält 82% Ausbeute.

### Beispiel 4

Durchführung wie Beispiel 3. Anstelle von Kaliumjodid wird 1 g Tetraethylammoniumjodid verwendet. Man erhält 78% Ausbeute.

### Beispiel 5

14,5g (0,1 mol) 2,2-Difluor-1-bromethan, 0,5g Kaliumjodid werden in einem Autoklaven mit 50g n-Butanol, der einen Wassergehalt von 250 ppm, d.h. 0,025 Vol.%, aufweist, vorgelegt und mit 6,8g Ammoniak versetzt. Das molare Verhältnis von 2,2-Difluor-1-bromethan zu Ammoniak beträgt 1 : 4. Die Reaktionsmischung wird auf 150 °C erhitzt und 2 Stunden bei dieser Temperatur gerührt. Die Reaktionsmischung wird abgekühlt auf 50°C und der überschüssige Ammoniak abdestilliert. Aus der Reaktionsmischung wird das Difluorethylamin anschließend bei 1-10 mbar herausdestilliert. Das Destillat, das noch geringe Mengen an Lösungsmittel enthält wird dann bei Normaldruck redestilliert. Man erhält 70% Ausbeute.

### Vergleichsbeispiel

14,5g (0,1 mol) 2,2-Difluor-1-bromethan, 1g Kaliumjodid werden in einem Autoklaven mit 50g DMSO vorgelegt und mit 27,5g einer 25-%igen wässrigen Ammoniaklösung versetzt. Die Reaktionsmischung wird auf 100°C erhitzt und 1,2 Stunden bei dieser Temperatur gerührt. Die Reaktionsmischung wird abgekühlt auf 50°C und der überschüssige Ammoniak abdestilliert. Aus der Reaktionsmischung wird das Difluorethylamin anschließend bei 1-10 mbar herausdestilliert. Das Destillat, das noch geringe Mengen an Lösungsmittel enthält wird dann bei Normaldruck redestilliert. Man erhält 13% Ausbeute.

## Patentansprüche

1. Verfahren zur Herstellung von 2,2-Difluorethylamin, umfassend
die Umsetzung von 2,2-Difluor-1-halogenethan mit der allgemeinen Formel (I):
CHF₂-CH₂Hal (I),
wobei Hal für Cl, Br, oder Jod steht,
mit Ammoniak in einem Lösungsmittel, das einen Wassergehalt von maximal 0,5 Vol.-% aufweist und in Gegenwart eines die Reaktion mit Ammoniak beschleunigenden Katalysators, wobei der Katalysator ausgewählt ist aus der Gruppe bestehend aus Alkalibromiden und -jodiden, Ammoniumbromid und Ammoniumjodid, Tetraalkylammoniumbromiden und -jodiden.

2. Verfahren nach Anspruch 1 , wobei das molare Verhältnis von 2,2-Difluor-1-halogenethan zum eingesetzten Ammoniak im Bereich von 0,8 : 1 bis 1 : 30 liegt.

3. Verfahren nach einem der Ansprüche 1 oder 2, wobei das Lösungsmittel ausgewählt ist unter Methanol, Ethanol, Isopropanol, Butanol, 2-(2-Ethoxyethoxy)-ethanol, Diethylenglykol, Ethylpropylether, Methyl-tert-butylether, *n*-Butylether, Anisol, Phenetol, Cyclohexylmethylether, Dimethylether, Diethylether, Dimethylglycol Diphenylether, Dipropylether, Diisopropylether, Di-*n*-butylether, Diisobutylether, Diisoamylether, Ethylenglycoldimethylether, Isopropylethylether, Methyl-tert-butylether, Diethylenglykoldimethylether, Triethylengylkoldimethylether, Tetrahydrofuran, Dioxan, und Polyether des Ethylenoxids und/oder Propylenoxid, Hexamethylenphosphorsäuretriamid, Formamid, N,N-Dimethyl-acetamid, *N*-Methyl-formamid, *N,N-*Dimethyl-formamid, *N,N* Dipropyl-formamid, *N,N-*Dibutyl-formamid, *N*-Methyl-pyrrolidin, *N-*Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N*'-1,4-Diformyl-piperazin und Dimethylsulfoxid.

4. Verfahren nach einem der Ansprüche 1 bis 3, wobei der Katalysator *in-situ* gebildet wird.

5. Verfahren nach einem Ansprüche 1 bis 3, wobei der Katalysator ausgewählt ist unter Kaliumbromid, Natriumjodid, Kaliumjodid, Tetrabutylammoniumbromid.

## Claims

1. Process for preparing 2,2-difluoroethylamine, comprising
the reaction of 2,2-difluoro-1-haloethane having the general formula (I):
CHF₂-CH₂Hal (I),
where Hal is Cl, Br, or iodine with ammonia in a solvent which has a maximum water content of 0.5% by volume and in the presence of a catalyst which accelerates the reaction with ammonia, wherein the catalyst is selected from the group consisting of alkali metal bromides and iodides, ammonium bromide and ammonium iodide, tetraalkylammonium bromides and iodides.

2. Process according to Claim 1, wherein the molar ratio of 2,2-difluoro-1-haloethane to the ammonia used is in the range from 0.8:1 to 1:30.

3. Process according to either of Claims 1 and 2, wherein the solvent is selected from methanol, ethanol, isopropanol, butanol, 2-(2-ethoxyethoxy)ethanol, diethylene glycol, ethyl propyl ether, methyl tert-butyl ether, *n*-butyl ether, anisole, phenetole, cyclohexyl methyl ether, dimethyl ether, diethyl ether, dimethylglycol, diphenyl ether, dipropyl ether, diisopropyl ether, di-*n*-butyl ether, diisobutyl ether, diisoamyl ether, ethylene glycol dimethyl ether, isopropylethyl ether, methyl tert-butyl ether, diethylene glycol dimethyl ether, triethylene glycol dimethyl ether, tetrahydrofuran, dioxane, and polyethers of ethylene oxide and/or propylene oxide, hexamethylenephosphoramide, formamide, N,N-dimethylacetamide, N-methylformamide, *N,N-*dimethylformamide, *N,N*-dipropyl-formamide, *N,N-*dibutylformamide, *N*-methylpyrrolidine, *N-*methylcaprolactam, 1,3-dimethyl-3,4,5,6-tetrahydro-2(1H)-pyrimidine, octylpyrrolidone, octylcaprolactam, 1,3-dimethyl-2-imidazolindione, *N*-formylpiperidine, *N,N*'-1,4-diformylpiperazine and dimethyl sulphoxide.

4. Process according to any of Claims 1 to 3, wherein the catalyst is formed *in situ.*

5. Process according to any of Claims 1 to 3, wherein the catalyst is selected from potassium bromide, sodium iodide, potassium iodide, tetrabutylammonium bromide.

## Revendications

1. Procédé de fabrication de 2,2-difluoroéthylamine, comprenant
la mise en réaction de 2,2-difluoro-1-halogénoéthane de formule générale (I) :
CHF₂-CH₂Hal (I),
Hal représentant Cl, Br ou iode, avec de l'ammoniac dans un solvant qui présente une teneur en eau d'au plus 0,5 % en volume et en présence d'un catalyseur accélérant la réaction avec l'ammoniac, le catalyseur étant choisi dans le groupe constitué par les bromures et iodures alcalins, le bromure d'ammonium et l'iodure d'ammonium, les bromures et iodures de tétraalkylammonium.

2. Procédé selon la revendication 1, dans lequel le rapport molaire entre le 2,2-difluoro-1-halogénoéthane et l'ammoniac utilisé se situe dans la plage allant de 0,8:1 à 1:30.

3. Procédé selon l'une quelconque des revendications 1 ou 2, dans lequel le solvant est choisi parmi le méthanol, l'éthanol, l'isopropanol, le butanol, le 2-(2-éthoxyéthoxy)-éthanol, le diéthylène glycol, l'éther éthylpropylique, l'éther de méthyle et de tert-butyle, l'éther n-butylique, l'anisole, le phénétol, l'éther cyclohexylméthylique, l'éther diméthylique, l'éther diéthylique, le diméthylglycol, l'éther diphénylique, l'éther dipropylique, l'éther diisopropylique, l'éther di-n-butylique, l'éther diisobutylique, l'éther diisoamylique, l'éther diméthylique d'éthylène glycol, l'éther isopropyléthylique, l'éther de méthyle et de tert-butyle, l'éther diméthylique de diéthylène glycol, l'éther diméthylique de triéthylène glycol, le tétrahydrofurane, le dioxane et les polyéthers d'oxyde d'éthylène et/ou d'oxyde de propylène, le triamide de l'acide hexaméthylènephosphorique, le formamide, le N,N-diméthyl-acétamide, le N-méthyl-formamide, le N,N-diméthyl-formamide, le N,N-dipropyl-formamide, le N,N-dibutyl-formamide, la N-méthyl-pyrrolidine, le N-méthyl-caprolactame, la 1,3-diméthyl-3,4,5,6-tétrahydro-2(1H)-pyrimidine, l'octylpyrrolidone, l'octylcaprolactame, la 1,3-diméthyl-2-imidazolinedione, la N-formyl-pipéridine, la N,N'-1,4-diformyl-pipérazine et le diméthylsulfoxyde.

4. Procédé selon l'une quelconque des revendications 1 à 3, dans lequel le catalyseur est formé in situ.

5. Procédé selon l'une quelconque des revendications 1 à 3, dans lequel le catalyseur est choisi parmi le bromure de potassium, l'iodure de sodium, l'iodure de potassium, le bromure de tétrabutylammonium.
